# EUROPEAN PATENT APPLICATION

(11) **EP 2 143 502 A1**
(43) Date of publication of application: **13.01.2010**
(21) Application number: 08012123.9
(22) Date of filing: 04.07.2008
(51) Int. Cl.: B05B 12/00

(54) **Process of labeling a space element in a space of air with a fragrance**

(71) Applicant: BrainInnova GmbH & Co. KG, 2314 Luxembourg (LU)
(72) Inventor: Morasch, Ludwig, 98000 Monaco (MC)
(74) Representative: Blodig, Wolfgang

(57) **Abstract**

A process of transiently labeling a space element of a space of air for a user of said space of air, whereby said space of air comprises at least a first and a second space element, said process comprising releasing a fragrance to the air of at least one of said space elements.

## Description

The present invention relates to a space of air such as a building or a parking garage having a high efficiency for users.

Parking garages, buildings or other spaces of air are widely used in urban settlements and elsewhere for use by many people, e.g. for allowing transient storage of cars. Parking garages and other buildings are frequently very big, and the efficiency by which such buildings can be used by users depends inter alia on the possibility and efficiency of users finding a desired area in such building. Parking garages may have several hundred parking spaces that may be located on different floors. When a user of a parking garage drives into a parking garage, he/she usually drives through a parking floor and, if necessary, through several parking floors until an empty parking space is found and used for parking a car. When the user later returns to the parking garage to find his car, users frequently have problems to recall the particular parking area or parking floor where they have left their car. Generally used numbering systems like an assignment of letters to parking floors is of no help, since users tend to forget the letter or number designating the parking area in which they have left their car. Users therefore frequently have to search several parking areas or several parking floors of a parking garage to find their car, which wastes valuable time of the user. Further, this wastes valuable time for the owner of the parking garage and for other people in need of a free parking space, since parking spaces remain occupied longer than necessary. Therefore, the overall efficiency of using conventional parking garages is low. Moreover, parking garages are generally all but attractive places, which makes lengthy searches for a parked car, notably late in the evening or for women, very unpleasant.

### Summary of the Invention

It is therefore an object of the invention to provide a space of air which allows a higher throughput of users by shortening the time required for finding a previously visited space element of the space of air. It is another object of the invention to provide a space of air such as a parking garage of improved commercial efficiency. It is another object of the invention to provide a space of air such as a parking garage which is more attractive for a user.

These objects are achieved by a process of labeling a space element in a space of air for a user of said space of air, whereby said space of air comprises at least a first and a second space element, said process comprising releasing a fragrance to the air of at least one of said space elements. Thereby, the fragrance present in the air of the space element labels said space element.

These objects are further achieved by a use of a fragrance in a space of air comprising at least a first and a second space element for labeling at least one of said space elements for users of said space of air. The fragrance is used for distinguishing said first space element from said second space element by a user of the space of air.

The invention also provides a building having at least two different space elements, wherein a first fragrance is present in the air of a first space element and a second fragrance different from said first fragrance is present in the air of a second space element of said building for labeling said first and second space elements differently.

The invention also provides a parking garage having at least two different parking areas, wherein a first fragrance is present in the air of a first parking area and a second fragrance different from said first fragrance is present in the air of a second parking area of said parking garage for labeling said first and second parking areas differently.

The invention further provides a process of running a parking garage, comprising informing users of the parking garage of the information content of a fragrance in parking areas of said parking garage, and allowing users to park a car in the parking garage.

It has been found that the use of a fragrance designating or labeling a space element in a space of air enables the user of a space of air to easily memorize and recall a space element that has been previously visited by the user. In the invention, one or more fragrances are used to affect the memory of a user without the user having to actively memorize the fragrance. In the case of a parking garage, if a user leaves his car in a particular parking area, the user is exposed to the fragrance in the air of the space element corresponding to the chosen parking area. The fragrance can be memorized unconsciously by the user. When the user later returns to the parking garage to find his car, parking areas having a fragrance different from that memorized by the user are immediately recognized as false, and the parking area having the memorized fragrance is recognized as right. This system provides guidance for the user in a building such as to the parked car in a parking garage.

The invention is based on the surprising finding that fragrances can be memorized easily by users. Therefore, fragrances may be used as an information carrier for providing guidance to users in spaces where orientation is difficult. Notably, fragrances may be used for labeling space elements of a space of air. The use of fragrances in the invention is universal, since no knowledge of a particular language is required for labeling or designating space elements in a space of air. Moreover, any subjective component in the perception of a fragrance among different users is eliminated, since the same subjective perception will apply to a particular user when a selected space of air is visited the first and a second time. Users of the space of air of the invention such as a parking garage need, on average, less time to find a previously visited space element. Thus, use of the space of air by a user is efficient. In the case of a parking garage and a parking process, use of the parking garage is efficient in that more users can use the parking garage of the invention during a predetermined period of time of e.g. one day. Further, the parking garage of the invention is more attractive and appealing to users, thus improving the turnover of the parking garage. As a further advantage, the fragrance used in the invention has a positive effect on the mood of users and is enjoyed by users, whereby users have a high tendency to reuse the space of air, whereby the profitability of the space of air is high.

### Detailed Description of the Invention

The space of air of the invention may be any outdoor or indoor space. Said space of air may or may not be enclosed by barriers such as walls that reduce or prevent exchange of air between within said space of air and the exterior of the space of air. Since a fragrance is released in the invention into the air of space elements of said space of air, it is advantageous if wind is low or reduced by suitable constructional means such as wind barriers or walls, so that said fragrance can be more easily kept in space elements of said space of air for a desired period of time.

Said space of air may be a train. In trains, it tends to be difficult for a user to find the area where his seat is located after having left the seat due to the uniformity of the interior of trains. Alternatively, said space of air may be a buidling, i.e. a space of air having air exchange barriers such as walls that provide a delimitation of the inside of said building from the outside of said building. However, walls of the building do not need to enclose the building completely. Instead, openings may be present in the walls e.g. for allowing entrance of light or air to the inside of the building. Said building may have one or more than one such as two, three or more story levels. In one embodiment, said building is a station such as a bus, train or underground station. In another embodiment, said building is a parking garage.

A space element is a three-dimensional portion of said space of air. Said space of air comprises at least a first and a second space element. Said space of air may have three, four or more space elements, as desired. If said space of air is a building, said first and said second space elements may be located on the same or on different story levels of the building. Plural space elements may or may not be separated from each other by a physical barrier such as a wall or a ceiling. For example, each story level may represent one space element of said building, whereby said space elements are separated by a ceiling. Alternatively or additionally, two or more space elements may be present on the same story level, whereby the space elements may or may not be separated by physical barriers such as walls. In a further embodiment, a wall may provide a partial separation of two space elements for reducing exchange of air between the space elements.

If said space of air is a parking garage, a space element, i.e. a parking area of the parking garage, may have at least one parking space. A parking space is a space for parking one car. A parking area may have more than one parking spaces, e.g. at least 2, at least 3, at least 5, at least 10, at least 20, at least 50 or at least 100 parking spaces.

In the invention, a fragrance is applied to the air of at least one of the space elements of the space of air. The fragrance must be applied or released such that it is present in the air of the space element. The fragrance contains one or more chemical compounds that cause an olfactory sensation in humans. It is not necessary that the olfactory sensation is caused in each and every human being. However, the olfactory sensation should be caused in the majority or all of the users of a given space of air. Further, it is not necessary that the fragrance causes the same qualitative or quantitative olfactory sensation in all users of the space of air. The chemical compound or compounds causing the olfactory sensation of the fragrance may be gasous, liquid or solid under standard conditions (25 °C, 101.3 MPa). In the case of a solid, the vapor pressue of the solid at standard conditions must be > 0. In order to be perceivable by the olfactory sensation, the fragrance must be applied such to the space elements that it is present in the air of said space element. Typically, said chemical compound or compounds is/are gasous under standard conditions, or they are liquid and are applied as an aerosol to the air of said of said space elements. Application of a chemical compound that is liquid at room temperature to the the air of a space element may for example be achieved by spraying the liquid compound or a solution of the liquid in a suitable solvent (e.g. water or a C1-4 alcohol) into the air. Application of a chemical compound that is solid at room temperature to the the air of said space element may for example be achieved by spraying a solution of the solid compound in a suitable solvent (e.g. water or a C1-4 alcohol) to the air. Spraying may involve use of a suitable carrier gas such as air, nitrogen, argon or carbon dioxide in the spraying process. With regard to the method of application of the fragrance to the air of a space element, the invention is not limited.

The labeling of a space element is transient in that the label can be largely removed by stopping the release of the fragrance in combination with exchange of the air in a space element by fresh air. However, the labeling of the invention requires that the label remains present over a predetermined period of time such that the space element can be recognized by a user that returns to the space of air after having been absent for a while. Typically, remaining present over a predetermined period of time of said labeling requires repeated release of said fragrance (see below). Thus, the fragrance is applied such that the labeling achieved by said fragrance remains or is renewed in the space element over a predetermined period of time. The duration of the predetermined period of time depends on the type of space of air and may depend on the average duration of absence of users from the space of air. In general, the fragrance is released such to a space element that the fragrance can be sensed by a user for at least one hour from the onset of fragrance release. In another embodiment, the fragrance can be sensed by a user for at least three hours, in a further embodiment for at least 5 hours, in a further embodiment for at least 7 hours from the onset of fragrance release into the air of a space element.

The dosage of the fragrance, i.e. the concentration of the fragrance in the air of the space element should be such that a pleasing odor is obtained. This dosage can be easily found by testing a selected space element of a given space of air using a selected spraying equipment. The concentration should be sufficiently low such that the fragrance is not unpleasant. The dosage of the fragrance as well as the release pattern (see below) may be adjusted depending on air movement, air exchange with the outside of said space of air, air convection, size of a space element, frequency of use of the space of air by users, average number of users in a space element, presence or amount of exhaust gases from combustion engines etc. Some chemical compounds that cause an olfactory sensation also cause a use effect, whereby the odor can no longer be sensed by humans after a certain period of uninterrupted exposure to a high concentration. This effect may be avoided by keeping the fragrance intensity sufficiently low. Further, the fragrance may be applied not to the entire space element but only to the entrance portion of a space element, whereby the exposure time of a user to the fragrance may be reduced while still allowing the user to identify the space element.

The fragrance may be released into the air of a space element continuously at a suitable rate. Alternatively, the fragrance may be released periodically at suitable intervals. Alternatively, the fragrance may be released periodically, whereby release is stopped after a first pulse of release and further pulses are released when the fragrance intensity in the air of a space element falls below a predetermined threshold. At least in the latter case, the release may be controlled by a control system comprising a sensor that measures the concentration of the fragrance in the air of a space element. The concentration of the fragrance in the air of the space element may for example be determined by measuring the refractive index of an air sample or by measuring the absorption behaviour of an air sample with respect to electromagnetic radiation such as uv, visible or infrared light. Alternatively, the remaining fragrance in an air sample may be qualitatively determined by a testing person using his olfactory sensation. Regarding the release pattern of the frangrance, the invention is not limited.

Said space of air may comprise a control system that controls release of a fragrance to at least one of said space elements depending on one or more parameters. Said control system comprises a sensor for measuring said parameter, a fragrance release device for releasing a fragrance to the air of a space element, and a control device that controls the fragrance release device dependent on a signal of the sensor that measures the parameter. Said control system may control release of a fragrance to a space element depending on many different parameters such as on one or more of the following group: concentration of the fragrance already present in the space element, air movement or air convection in a space element, air exchange with the outside of said space of air, frequency of use of a space element by users, average number of users in a space element, movement by users in a space element, presence or amount of exhaust gases from combustion engines in a space element etc. Methods of meauring these parameters are known. For example, in order to determine the presence of users in a space element, a motion detector may be used. In the absence of movement in a space element as determined by the movement detector, the control system may reduce or stop release of the fragrance to the space element. The control system may resume release of a fragrance when a user approaches or enters the space element.

The space of air may comprise a separate control system as described above for each space element to be provided with a fragrance. Thus, each of said at least first and second space element may have a control system.

The control system of the invention may additionally respond to a signal received by the control device from a sender (e.g. a mobile telephone, a transmitter, a transponder), whereby the control device may control release of a particular fragrance corresponding to the signal received from the sender. The sender may be carried by a user of the space of air or may be incorporated in a car if the space of air is a parking garage. This embodiment allows an individualised service to particular users of the space of air. For example, a particular parking space rented by a user may be supplied with a fragrance selected by the user by inputting information on the selected fragrance at the sender and transmitting the information to the control device by the sender when the user approaches his parking space.

Many fragrances useful for the invention are available commercially. Examples of fragrances are extracts from plants such as from rose, lilac, jasmine, osmanthus, vanilla, mimosa, tuberose, blossoms of citrus or ylang-ylang trees etc. Further examples are extracts from animals such as ambergris, castoreum, honeycomb, musk etc. Instead or fragrances from natural sources, synthetic fragrances may be used. Examples of chemical compounds usable in fragrances of the invention are cinnamic aldehyde, coumarin, limonene, eugenol etc. Many flagrances are described in Ullmann's Encyclopedia of Industrial Chemistry, 6th edition, vol. 14, pages 73-199, Wiley-VCH Verlag GmbH & Co. KGaA, Weinheim, 2003, which is incorporated by reference herein in its entirety.

If the space of air has only two space elements, it may be sufficient to apply a flagrance to only one of said space elements, which may be sufficient for distinguishing these two space elements as odorous and non-odorous. If two or more different fragrances are released to two or more different space elements, the fragrances should be sufficiently different such that users of the space of air can distinguish the two or more fragrances. The space of air may have three, four or more space elements, each being provided with a different flagrance, i.e. three, four or more different flagrances, respectively. The difference between two different fragrances should be sufficiently big to avoid confusion of the different fragrances by a user.

Herein, a user is a human being using said space of air.

The process of the invention may comprise, as a first step, informing a user of the information encoded by the fragrance or fragrances used in a space of air, followed by the use of the space of air by said user.

The process and use of the invention are, in one embodiment, carried out in a parking garage for helping users of the parking garage to efficiently identify a parking area where the user has left his car. If said space of air is a parking garage, said space elements are parking areas in said parking garage. A parking area may comprise more than 100 parking spaces for cars. Alternatively, a parking area may have up to 50 parking areas.

Combination of the invention with the parking garage of WO 2006/63692
In one embodiment, the invention may be combined with the parking garage described in WO 2006/63692. In this embodiment, the parking garage may have at least two parking areas inside said parking garage, each parking area comprising a plurality of parking spaces adjacent to a wall, a wall segment of said wall extending along said plurality of parking spaces, said wall segment having a color pattern comprising at least two contrasting colors, whereby the color patterns of wall segments of different parking areas are different and preferably differ in color and in the pattern of the color patterns. Further, at least one parking area is provided with a fragrance for labeling the parking area as described above. Moreover, a parking area may have a conrol system as described above.

Each parking area may comprise a plurality of parking spaces adjacent to a wall, whereby a segment of said wall extends, in horizontal direction, along said plurality of parking spaces. Said plurality of parking spaces comprises at least two parking spaces, preferably at least three parking spaces, more preferably at least four parking spaces. There is no strict upper limit for the number of parking spaces of said plurality of parking spaces that are located adjacent to said wall. This upper limit is generally determined by the length of said wall segment and may be more than 100. Preferably, the upper limit is 50 parking spaces. Herein, a parking space is considered to be located adjacent to a wall, if the boundaries of said parking space are within 2 meters, preferably within 1 meter from said wall. Said boundaries may be signs or markers indicating the location of a parking space such as lines drawn on the floor. If no such boundaries are present, a parking space is considered to be adjacent to a wall if a car can be parked within 2 meters, preferably within 1 meter from said wall. The number of parking spaces in a plurality of parking spaces of different parking areas may be the same or may be different.

Not all parking spaces of a parking area have to be located adjacent to a wall, provided that a plurality of parking spaces is located adjacent to a wall. Preferably, said wall segment carrying the color pattern should be viewable essentially from all parking spaces of the corresponding parking area. The minimum number of parking spaces per parking area is 2. The maximum number of parking spaces per parking area is not limited. However, the maximum number of parking spaces in a parking area should be such to provide effective guidance for a user to his car. Therefore, the maximum number of parking spaces in a parking area should be at most 200, preferably at most 100, more preferably at most 50 parking spaces.

Said wall may be an outside wall of said parking garage or an inside wall of said parking garage. Said wall serves at least the purpose of carrying said color pattern. Said wall may be made of concrete, of brickwork, of gypsum panels or any other material providing a solid support for said colar pattern. Said wall typically extends from the bottom of a parking floor up to the ceiling. Supporting walls are typically made of reinforced concrete or brickwork, whereby non-supporting walls may be made of less stable materials like gypsum panels.

The segment of a wall extends along said plurality of parking spaces. Extending along said plurality of parking spaces forces a user of a parking space of said plurality of parking spaces to get in close proximity to said color pattern on said wall. A segment of said wall is defined as having one type of color pattern thereon. A segment of a wall may have the same color pattern repeatedly several times next to each other. A wall of said parking garage may comprise more than one wall segment, whereby the color patterns of said wall segments will be different. It is not necessary, that all parking spaces of a particular parking area are located adjacent to the wall, provided that a plurality of parking spaces of a parking area are located adjacent to a wall. It is the plurality of parking spaces that is located adjacent to a wall, which defines said parking area. Thus, each of said at least two parking areas has one wall segment. Different wall segments carry different color patterns and can thus easily be assigned by a user of the parking garage to the parking area where he or she has left his car.

The color pattern of each wall segment comprises at least two contrasting colors. In order to give an easily memorizable color pattern, at least 40, preferably at least 50, more preferably at least 60, and most preferably at least 70 % of the surface of a selected wall segment should be covered by a chromatic color. "Chromatic" means a color not lying on the vertical lightness axis in the color solid, i.e. not an achromatic color. Preferably, a chromatic color is one having a chroma in the CIELab system of at least 20. In calculating the surface area of a wall segment, the total height of said wall from top to bottom is used in vertical direction, i.e. the height from the ground to the ceiling of a floor (story) of the parking garage. In horizontal direction, the length of the wall segment along a plurality of parking spaces is used. This length is at least the sum of the breadth of two parking spaces, i.e. about 3.5 m. Said chromatic color has a chroma in the CIELab system of at least 20, preferably at least 30, more preferably at least 40. Preferably, the lightness of the chromatic color covering at least 40 (preferably at least 50, 60, or 70) % of the surface of said wall segment is between 20 and 90, preferably between 30 and 85, more preferably between 40 and 85, and most preferably between 50 and 80 in the CIELab system. The CIELab system used herein is the CIE 1976 system. See also: CIE: Colorimetry, 2nd ed., CIE, Vienna, 1986. Further, chapters 3.3, 3.4, and 3.5 of volume 11 of Ullman's Encyclopedia of Industrial Chemistry, 6th edition, Wiley-VCH, 2003, are incorporated herein by reference.

In said color pattern, said at least two contrasting colors alternate to produce a pattern of said contrasting colors. "Alternate" means that said at least two different colors cover different parts of the surface of said wall segment, i.e. are not (exclusively) mixed and not (exclusively) used on top of each other to give a single color impression. The arrangement of wall surface areas covered by different contrasting colors relative to each other defines the pattern of the color pattern. The colors of said color pattern may be arranged in any way. Said pattern may comprise any combination of straight and curved dividing lines between surface areas of different contrasting colors. Thus, a color pattern is defined herein by the contrasting colors used and by the patterns formed by said contrasting colors. Examples of color patterns according to the invention are numbers, letters, words, symbols or any combination of these in a first color on a background having a second color, whereby the first and the second color are contrasting colors.

In a preferred embodiment, the color pattern is the logo of a company on the background of a corporate identity color of said company. Most preferably, the color pattern is the logo of a company well-known at the location of the parking garage. In this case, the best effect on the memory of the parking garage user can be obtained, since this produces an association in the user's memory of a famous name or company with the parking area where the user has left his car. Such an association enables the user to easily recall the color pattern of the used parking area.

In order to be contrasting, said contrasting colors differ in at least one property selected from lightness, hue and chroma. More preferably, said contrasting colors of a color pattern differ in hue and in chroma. Most preferably, said at least two different colors differ in lightness, hue and in chroma. Differences in hue should be at least 30°, preferably at least 50°, more preferably at least 70°, and most preferably at least 90° in the a-b-plane of the CIELab color space. Differences in chroma should be at least 20, preferably at least 30, more preferably at least 40, and most preferably at least 50.

Also preferred are embodiments wherein:
said two contrasting colors differ in hue by at least 50° and in chroma by at least 20;
said two contrasting colors differ in hue by at least 70° and in chroma by at least 20;
said two contrasting colors differ in hue by at least 70° and in chroma by at least 30;
said two contrasting colors differ in hue by at least 50°, in chroma by at least 30, and in lightness by at least 30. These embodiments may apply to one, to two or to more different color patterns.

Said different contrasting colors of a selected color pattern are chromatic colors. In another embodiment, said color pattern comprises three, four or more contrasting colors.

If the parking garage of the invention comprises at least two parking areas each having a wall segment having a color pattern, the parking garage has at least two color pattern that are different. The parking garage may have three, four, or more parking areas, each having a wall segment with a color pattern, whereby all color patterns are different.

The difference between said color patterns is preferably in color and in the pattern formed by said at least two contrasting colors. Difference in color means that a first color pattern has at a selected point a color different from the color at a corresponding point of a second color pattern. Difference in pattern means that the arrangement of colors of different color patterns is different. In general, two color patterns may differ either in color or in pattern, or in color and in pattern. In the present invention, two color patterns differ most preferably in color and in pattern.

Herein, a first color pattern differing in color from a second color pattern preferably has at least one color essentially absent in the second color pattern. A color not present in another color pattern preferably covers at least 10, preferably at least 20, more preferably at least 30, and most preferably at least 40 % of the surface area of the wall segment carrying said color pattern. "Essentially absent" means that the essentially absent color covers at most 5% of the surface area of wall segment.

Preferred embodiments of different color patterns are the following: a color of a first color pattern covers at least 40% of the surface area of a first wall segment and a different color of a second color pattern covers at least 40% of the surface area of a second wall segment, whereby said color of said first color pattern and said color of said second color pattern differ in hue by at least by 50° or in chroma by at least 30; more preferably, said colors differ in hue by at least 70° or in chroma by at least 40; even more preferably, said colors differ in hue by at least 50° and in chroma by at least 20; even more preferably, said colors differ in hue by at least 70° and in chroma by at least 20; even more preferably, said colors differ in hue by at least 70° and in chroma by at least 30; most preferably, the colors of the previous embodiments each covers more than 50% of the surface area of the respective wall segments.

The ratio of the repetition period of a color pattern according to the invention on its wall segment to the repetition period of the parking spaces adjacent to said wall are preferably between 0.25 to 10, preferably 0.5 to 5. A ratio of 0.25 corresponds to a situation where a color pattern is repeated four times on a length of said wall (in horizontal direction) corresponding to the breadth of one parking space (measured parallel to said wall). A ratio of ten corresponds to the situation of one color pattern that extends over a length corresponding to ten parking spaces without repeating said color pattern over this length. Thus, said ratio is a measure of the number of a repetitions of a color pattern on said wall segment per parking space adjacent to said wall. For the purpose of calculating said ratio on an absolute scale, the size of a parking space may be assumed to be 1.75 m for parking spaces arranged orthogonal to the adjacent wall. For parking spaces arranged parallel to the adjacent wall, the size of a parking space may be assumed to be 4 m. The orientation of a parking space relative to said wall may be such that the longer extension of the parking space may be parallel or orthogonal to the direction of said wall. In any case, the size of a parking space is always measured in parallel to said wall.

A selected color pattern is present at least once over a length of the corresponding wall segment, which gives a ratio of 2 if said plurality of parking spaces consists of 2 parking spaces and a ratio of 10 if said plurality of parking spaces consists of 10 parking spaces. A selected color pattern may be repeated once, twice, three times or more times over the length of the corresponding wall segment, i.e. be present two times, three times, four or more times, respectively, over a length of the corresponding wall segment. If a color pattern is repeated once over the length of the corresponding wall segment, said ratio is 1 if said plurality of parking spaces consists of 2 parking spaces and said ratio is 5 if said plurality of parking spaces consists of 10 parking spaces. Preferably, said ratio of the repetition period of said color pattern to the repetition period of the parking spaces adjacent to said wall is 0.5 to 5, more preferably 1 to 5, most preferably 2 to 5. The ratio of wall segments in different parking areas may be the same or may be different.

There are no limitations regarding the mode of application of the colors forming said color patterns on said wall segments. In one embodiment, said color patterns are painted on panels, e.g. gypsum panels, and the painted panels are mounted on said wall segment. In another embodiment, said color patterns are applied or printed on a sheet (e.g. of paper or plastic) and said sheet are then applied (e.g. glued) on said wall segments.

The paint used for painting or printing said color pattern may contain fluorescent dyes or may contain reflecting particles for providing the color pattern with an appealing look. These embodiment should be combined with an illuminating system illuminating said color patterns.

Said parking garage may have said color patterns not only on a wall but also on the floor and/or on the ceiling adjacent to the wall carrying said color pattern for improving the effect of the color pattern on the user of the parking garage. For the same purpose, the parking garage should comprise a lighting installation capable of illuminating the at least two color patterns.

The fragrance and the color patterns used in the parking garage of the invention may be suitably adjusted to each other in order to induce a particularly strong memorisation by users. For example, the fragrance and colors of the color pattern used in a parking area may be mutually adjusted to provoke a similar sensation by users. For example, a rose fragrance may be combined with a color pattern reminding users of roses. A color pattern may remind users of roses by containing colors in the color pattern reminding users of roses, e.g. red. Alternatively, the color pattern may be or may contain images of roses.

### Preferred embodiments

A process of transiently labeling a space element in a space of air for a user of said space of air, whereby said space of air comprises at least a first and a second space element, said process comprising releasing a fragrance to the air of at least one of said space elements such that said fragrance can be sensed by a user for at least three hours after the onset of releasing the fragrance.

Use of a fragrance in a parking garage, said parking garage comprising at least a first and a second parking area, for labeling at least one of said parking areas for distinguishing said first and said second parking area for users of said parking garage.

Building having at least two different space elements, wherein a first fragrance is present in the air of a first space element and a second fragrance different from said first fragrance is present in the air of a second space element of said building for labeling said first and second space elements differently, said building comprising a control system that controls release of a fragrance to at least one of said space elements, said control system comprises a sensor for measuring a parameter that correlates to the amount of fragrance required in a space element, a fragrance release device for releasing a fragrance to the air of a space element, and a control device that controls the fragrance release device dependent on a signal provided by said sensor to the control device.

### Brief description of the drawings

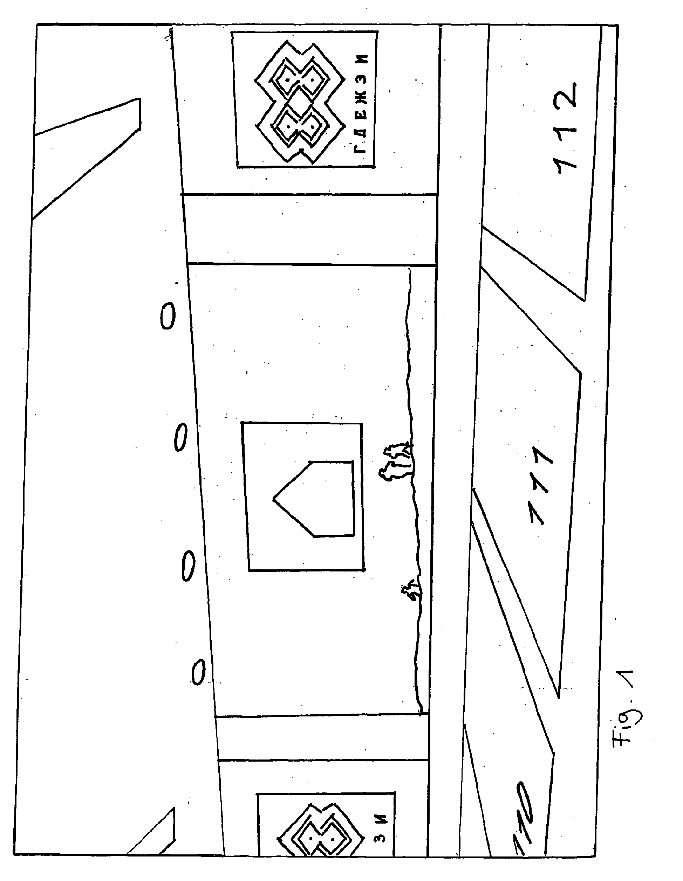

Fig. 1 shows a view on a sidewall in a parking garage. Fig. 1 indicates 3 parking areas, each parking area having a plurality of parking spaces (shown partially). The parking area in the middle of the figure consists of two parking spaces numbered as 110 and 111. The plurality of parking spaces 110 and 111 are located adjacent to a sidewall. The wall segment belonging to the parking area in the middle of the figure extends along parking spaces 110 and 111. The wall segment extending along parking spaces 110 and 111 has a color pattern having at least three contrasting colors, namely green for the lawn in the foreground, blue for the sky and white for the clouds. The blue color of the sky covers at least 40% of the surface of said wall segment and has a lightness of at least 50 and a chroma of at least 30 in the CIELab system. The color pattern further comprises a symbol in the form of an upwardly directed arrow and the words "CAR SPACE". The parking garage further has a light installation comprising four lights above the central wall segment, said light installation illuminating the color pattern under said light, whereby the color pattern can easily be seen by users of the parking area. Fig. 1 further shows two further parking areas flanking the central parking area on the left and on the right. These further parking areas have, on the same wall, wall segments extending along the respective parking spaces (partially shown) and color patterns (partially shown) on the respective wall segments. The color patterns of the three color patterns shown in Fig. 1 are different.

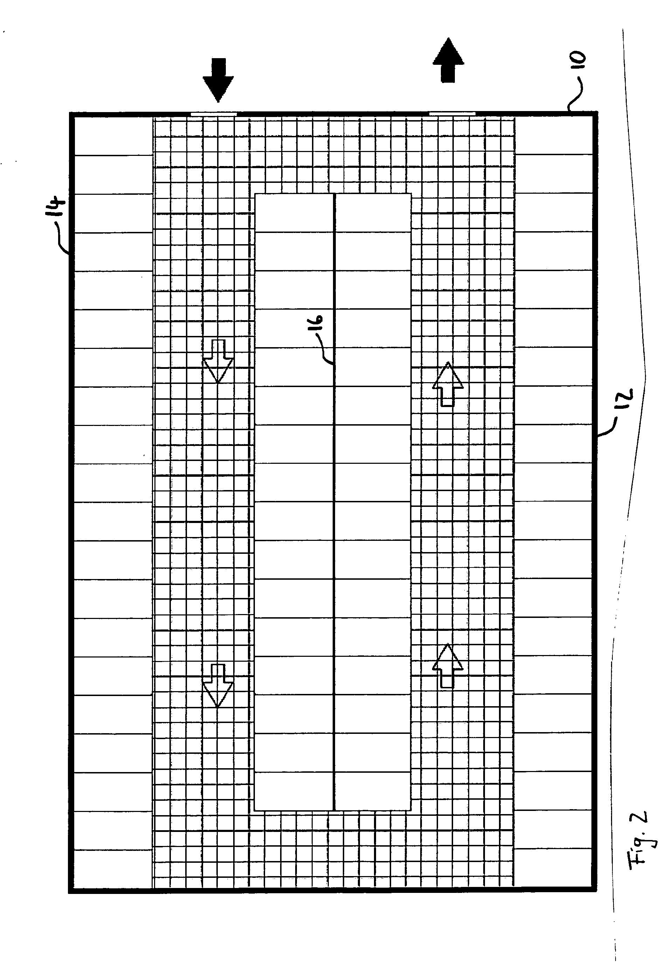

Fig. 2 shows a plan view on a parking floor of a parking garage according to the invention. Filled arrows indicate entrance and exit, respectively, to the parking floor. The checky area indicates the driving route for cars entering or leaving the parking floor, whereby the driving direction is indicated by arrows. Rectangular fields stand for parking spaces. 10 indicates a supporting sidewall of the parking garage. 12 indicates a first wall segment of said side wall, said wall segment extending along 20 parking spaces. 14 indicates a second wall segment of said sidewall, said wall segment extending along 20 parking spaces. 16 indicates a non-supporting sidewall extending in vertical direction from top to bottom of the parking floor. The parking floor shown in Fig. 2 is divided into two parking areas by sidewall 16. Sidewall 16 serves as a wall segment for the first parking area with its surface directed towards wall segment 12. Sidewall 16 also serves as a wall segment for the second parking area with its surface directed towards wall segment 14. The surfaces of wall segment 12 and the surface of sidewall 16 directed towards wall segment 12 carry a first color pattern (not shown). The surfaces of wall segment 14 and the surface of sidewall 16 directed towards wall segment 14 carry a second color pattern (not shown). In each parking area, the respective color pattern may be repeated several times. A color pattern may extend along 4 parking spaces and will be present 4 times on sidewall 16 and 5 times on wall segments 12 and 14.

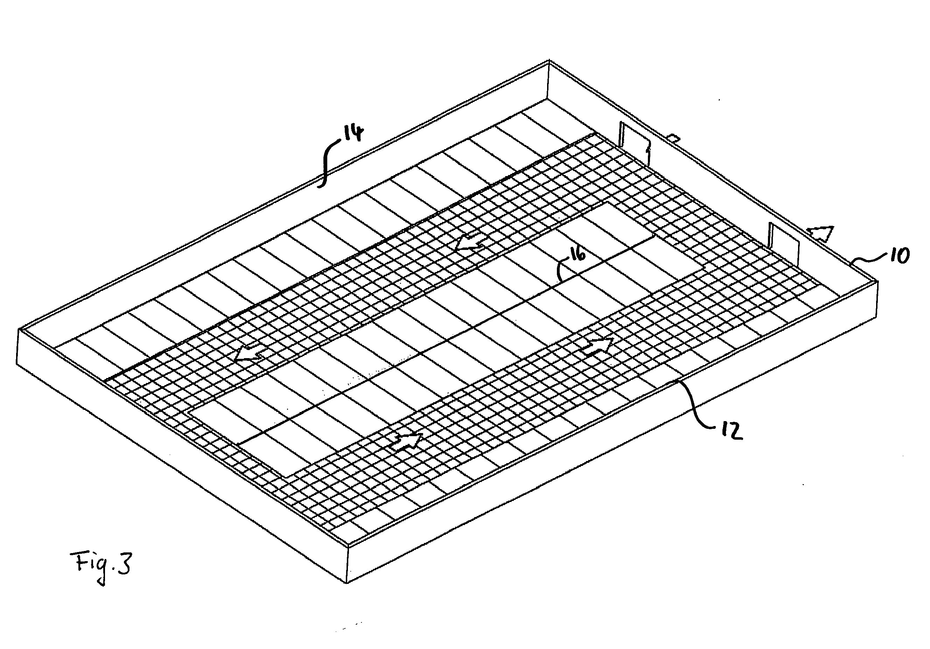

Fig. 3 is a perspective view into the parking floor shown in Fig. 2.

## Claims

1. A process of transiently labeling a space element of a space of air for a user of said space of air, whereby said space of air comprises at least a first and a second space element, said process comprising releasing a fragrance to the air of at least one of said space elements.

2. The process according to claim 1, wherein a first fragrance is applied to the air of said first space element and a second fragrance different from said first fragrance is applied to the air of said second space element.

3. The process according to claim 1, wherein said fragrance encodes an information allowing recognition and/or differentiation of said first and said second space element of said space of air by said user.

4. The process according to claim 1, wherein said fragrance is released as a gas or as an aerosol to the air of said space element.

5. The process according to claim 1, wherein said space of air is a train or a building such as a bus, underground or train station, or said building is a parking garage.

6. The process according to any one of claims 1 to 4, wherein said space of air is a parking garage and said first and second space elements are first and second parking areas, respectively, of said parking garage.

7. The process according to claim 6, wherein each parking area comprises a plurality of parking spaces adjacent to a wall, a wall segment of said wall extending along said plurality of parking spaces, said wall segment having a color pattern comprising at least two contrasting colors, whereby the color patterns of wall segments of different parking areas differ in color and in the pattern of the color patterns.

8. The process according to claim 7, wherein said two contrasting colors of a selected wall segment differ at least in hue and in chroma.

9. The process according to claim 7 or 8, wherein at least 40% of the surface of a wall segment is covered by color having a chroma of at least 20, preferably at least 30, in the CIELab system.

10. Use of a fragrance in a space of air, said space of air comprising at least a first and a second space element, for labeling at least one of said space elements for users of said space of air.

11. Building having at least two different space elements, wherein a first fragrance is present in the air of a first space element and a second fragrance different from said first fragrance is present in the air of a second space element of said building for labeling said first and second space elements differently.

12. Parking garage having at least two different parking areas, wherein a first fragrance is present in the air of a first parking area and a second fragrance different from said first fragrance is present in the air of a second parking area of said parking garage for labeling said first and second parking areas differently.

13. Parking garage according to claim 12, wherein each parking area comprises a plurality of parking spaces adjacent to a wall, a wall segment of said wall extending along said plurality of parking spaces, said wall segment having a color pattern comprising at least two contrasting colors, whereby the color patterns of wall segments of different parking areas differ in color and in the pattern of the color patterns.

14. The parking garage according to any of claims 12 or 13, wherein at least 40% of the surface of a wall segment is covered by color having a chroma of at least 20, preferably at least 30, in the CIELab system.

15. The parking garage according to any of claims 12 to 14, wherein a first color pattern of a first parking area comprises at least one chromatic color that is essentially absent in a second color pattern of a second parking area, said chromatic color covering at least 10 %, preferably at least 20 % of the surface area of the wall segment carrying said first color pattern.
